# EUROPÄISCHE PATENTSCHRIFT

(11) **EP 0 734 274 B1**
(45) Veröffentlichungstag und Bekanntmachung des Hinweises auf die Patenterteilung: **11.06.1997**
(21) Anmeldenummer: 95903333.3
(22) Anmeldetag: 09.12.1994
(51) Int. Cl.: A61M 36/00, A61N 5/10

(54) **VORRICHTUNG FÜR DIE MEDIZINISCHE STRAHLENTHERAPIE**
RADIATION-THERAPY DEVICE
DISPOSITIF DE RADIOTHERAPIE MEDICALE

(30) Priorität: 14.12.1993 DE 4342589
(43) Veröffentlichungstag der Anmeldung: 02.10.1996
(73) Patentinhaber: B.V. Optische Industrie "De Oude Delft", 2624 BC Delft (NL)
(72) Erfinder: NANKO, Norbert, D-79108 Freiburg (DE)
(74) Vertreter: Schmitt, Hans, Dipl.-Ing.
(86) Internationale Anmeldenummer: EP9404106
(87) Internationale Veröffentlichungsnummer: WO9516489

(56) Entgegenhaltungen:
- EP-A- 0 292 630
- AT-B- 390 884
- US-A- 5 030 195

## Beschreibung

Die Erfindung bezieht sich auf eine Vorrichtung für die medizinische Strahlentherapie mit einer im Behandlungsbereich beim Patienten anbringbaren, verformbaren Trägermatte für Führungshülsen, in denen eine im wesentlichen punktförmige Strahlenquelle verschiebbar und positionierbar ist, wobei die Trägermatte innerhalb ihrer Mattenebene verlaufende, vorgefertigte Aufnahmekanäle zur Aufnahme der Führungshülsen oder dergleichen aufweist.
Bei der Strahlentherapie ist das sogenannte Nachladeverfahren (Afterloading-Technik) bereits bekannt, bei dem ein röhrchenförmiger, durch eine Führungshülse gebildeter Applikator zunächst in eine gewünschte Position bei der zu behandelnden Stelle gebracht wird. Anschließend wird ein radioaktives Präparat als Strahlenquelle aus einem Stahlenschutzbehälter durch eine mit dem Applikator gekoppelte Hohlsonde ferngesteuert an die Behandlungsstelle in den Applikator geschoben.
Als Halterung für mehrere Führungshülsen oder Applikatoren sind Moulagen aus plastisch verformbarem Material, zum Beispiel aus Plastilin, Schaumgummi oder ähnlichem bekannt, in die nadelförmige Führungshülsen eingestochen werden. An die äußeren Enden der Führungshülsen sind Hohlsonden ankuppelbar, die mit dem Afterloading-Gerät verbunden sind und über das die in dem Strahlenschutztresor befindliche Strahlenquelle nacheinander in die einzelnen Führungshülsen (Applikatoren) einschiebbar sind.
Für eine gezielte Therapie ist es erforderlich, die Führungshülse innerhalb der Trägermatte (Moulage) genau zu positionieren. Dabei ist zu beachten, daß die Strahlenintensität nicht linear mit dem Abstand zur Strahlenquelle abnimmt, sondern daß sich, bedingt durch Sekundärstrahlung, in einem ganz bestimmten Abstand von der Strahlenquelle ein Intensitätsmaximum ergibt.
Zur Berechnung der Strahlendosis ist somit einerseits die Lage der Moulage selbst relativ zum Behandlungsfeld und andererseits auch die Lage der Führungshülsen innerhalb der Trägermatte von Bedeutung.
Beim Einstechen der aus Kunststoff bestehenden Führungshülsen in die Trägermatte besteht das Problem, daß ein gerader Verlauf der Führungshülsen und ein exaktes Einhalten eines gleichbleibenden Abstandes zu der Trägermattenoberfläche und auch zu benachbarten Führungshülsen praktisch nicht realisierbar ist.
Um ein seitliches Ausweichen der Führungshülsen beim Einstechen in die Trägermatte in Grenzen zu halten, werden für den Einstechvorgang Stahlmandrins in die Führungshülsen eingeschoben, um eine etwas höhere Biegesteifigkeit zu erzielen. Trotzdem ist bei dem manuellen Einstechvorgang eine exakte Lageeinhaltung der Führungshülsen meist nicht in dem erwünschten Maße möglich.

Ein weiteres Problem ergibt sich daraus, daß die Länge der Führungshülsen wegen des mit der Länge zunehmend schwieriger werdenden Einstechvorganges so kurz wie möglich sein soll und deshalb im wesentlichen auf die Größe der Trägermatte abgestimmt ist. Die Anschlüsse für die zum Afterloading-Gerät führenden Hohlsonden liegen somit nahe an der Trägermatte und damit bei einer Behandlung im Körperinneren ungünstig auch in einem Bereich, wo die Kupplungsstellen mit Körperflüssigkeit, Blut und dergleichen in Berührung kommen können.

Aus der EP 292 630 A1 kennt man bereits eine Vorrichtung für die medizinische Strahlentherapie, die innerhalb ihrer Mattenebene verlaufende, vorgefertigte Aufnahmekanäle für Führungshülsen aufweist, so daß dementsprechend auch eine exakte Lagevorgabe für eine Strahlenquelle möglich ist.
Die hierbei verwendeten Trägermatten sind als Einmalprodukte konzipiert und bestehen aus absorbierbaren Material. Sie weisen eine nur sehr geringe Dicke auf.
Wie bereits vorerwähnt, ist jedoch zu beachten, daß die Strahlenintensität nicht linear mit dem Abstand zur Strahlenquelle abnimmt, sondern daß in einem ganz bestimmten Abstand von der Strahlenquelle ein Intensitätsmaximum vorhanden ist.
Ein typischer (Minimal)Abstand der Strahlenquelle vom Behandlungsort beträgt etwa 5mm, so daß die zur Führung, aber auch als Abstandhalter dienende Trägermatte eine entsprechende Dicke von beispielsweise etwa 10mm haben muß.
Eine 10mm dicke Matte läßt sich aber an gekrümmte Bereiche schlecht oder nicht mehr genügend formanpassen, so daß der durch die Matte selbst vorgegebene Abstand zu dem zu bestrahlenden Bereich nicht immer eingehalten wird.
Aufgabe der vorliegenden Erfindung ist es, eine Vorrichtung der eingangs erwähnten Art zur Strahlentherapie zu schaffen, bei der eine exakte Vorausberechnung der zu verabreichenden Strahlendosis auch mit guter Genauigkeit in die Praxis umsetzbar ist. Die Trägermatte soll dazu trotz der strahlenphysikalisch bedingten Vorgabe einer vergleichsweise großen Dicke auch an vergleichsweise stark gekrümmte Bereiche anpassbar sein. Schließlich soll die Trägermatte wiederverwendbar einsetzbar sein.

Zur Lösung dieser Aufgabe wird erfindungsgemäß insbesondere vorgeschlagen, daß die Trägermatte einstückig aus Silikon oder einem silikonähnlichen, autoklavierbaren Material besteht, daß die Trägermatte aus einer Vielzahl von nebeneinander angeordneten, über Halsabschnitte miteinander verbundenen Kugeln oder gerundeten, kugelähnlichen, ballförmigen Körpern gebildet ist und daß die Aufnahmekanäle durch die Kugeln und/ die Halsabschnitte verlaufen.
Die Verformbarkeit der Trägermatte ist dadurch ganz erheblich verbessert und außerdem ergeben sich dadurch nur punktuelle Auflagen auf dem zu behandelnden Gewebe, so daß insbesondere bei Behandlung im Körperinneren ein unerwünschtes Anhaften vermieden wird.
Durch das verwendete Material für die Trägermatte ist diese einerseits hochflexibel und andererseits besteht dadurch die Möglichkeit, die Trägermatte nach Gebrauch in einem Autoklaven zu sterilisieren. Somit ist die Trägermatte wiederverwendbar und damit kostengünstig einsetzbar.

Die für die besonders gute Verformbarkeit der Trägermatte vorgesehene gerundete Form oder Kugelform der Einzelelemente trägt durch die glatten, nischenfreien Flächen auch noch in vorteilhafter Weise mit zu einer einfachen Sterilisierbarkeit der Trägermatte bei.
Schließlich läßt sich die Trägermatte vergleichsweise einfach zum Beispiel in einem Gießverfahren herstellen.
Durch die innerhalb der Mattenebene der Trägermatte verlaufenden, vorgefertigte Aufnahmekanäle zur Aufnahme oder zum Einstecken von Führungshülsen oder dergleichen besteht in vorteilhafter Weise die Möglichkeit, die Führungshülsen problemlos an den Stellen der Trägermatte jeweils in einem vorhandenen, in seiner Lage beziehungsweise seinem Verlauf exakt vorgegebenen Aufnahmekanal einschieben zu können.
Dementsprechend ist dann auch die eingeschobene Führungshülse exakt innerhalb der Trägermatte verlegt, so daß aufgrund dieser Lage eine genaue Strahlendosierung vorausberechenbar ist. Die Verwendung eines Mandrins als Hilfsmittel beim Einführen der Führungshülsen ist hierbei nicht erforderlich. Auch spielt die Länge der Führungshülsen keine Rolle mehr, so daß diese in ihrer Länge so dimensioniert werden können, daß ihre äußeren Anschlußenden für die Hohlsonde(n) an einer gut zugänglichen Stelle liegen.

Eine vorteilhafte Ausgestaltung der Erfindung sieht vor, daß die Aufnahmekanäle in einem vorgegebenen Raster angeordnet sind und daß vorzugsweise die Aufnahmekanäle parallel nebeneinander mit insbesondere gleichen Seitenabständen zueinander angeordnet sind.
Beim Bestücken der in der Trägermatte vorhandenen Aufnahmekanäle mit Führungshülsen hat man bei einer solchen Rasteranordnung eine ausreichende Auswahl für ein individuell angepaßtes Bestückungsmuster, so daß für die meisten Anwendungsfälle eine Universal-Trägermatte einsetzbar beziehungsweise verwendbar ist.

Eine weitere Ausgestaltung der Erfindung sieht vor, daß zumindest ein Teil der Aufnahmekanäle bezüglich der zu den Flachseiten der Trägermatte parallel verlaufenden Mittelebene versetzt angeordnet sind.
Sind beispielsweise alle Aufnahmekanäle zur Mittelebene versetzt, also näher an der einen Flachseite als an der gegenüberliegenden Flachseite angeordnet, so lassen sich durch Wenden der Trägermatte unterschiedliche Abstände zu dem zu behandelnden Gewebe einstellen. Bei einer im Querschnitt zickzackförmigen Anordnung der Aufnahmekanäle insbesondere beidseitig der Mittelebene lassen sich im Verlauf der Trägermatte unterschiedliche Bestrahlungsabstände einstellen.

Nach einer Weiterbildung der Erfindung ist vorgesehen, daß die Trägermatte Durchbrüche vorzugsweise zwischen den Aufnahmekanälen beziehungsweise den Kugeln oder dergleichen aufweist. Dadurch wird die Verformbarkeit und Anpaßbarkeit der Trägermatte verbessert und es sind damit auch Anwendungen unter beengten Verhältnissen und bei Auflage der Matte auf stark gewölbten Bereichen möglich.
Eine bevorzugte Ausführungsform der Erfindung sieht vor, daß die Kugeln an ihren einander zugewandten Seiten im Bereich der Verbindungsstellen mit den Halsabschnitten jeweils um die halbe Länge der Halsabschnitte abgeflacht sind und daß der Mittenabstand benachbarter Kugeln ihrem Durchmesser entspricht.
Dadurch kann der seitliche Rasterabstand benachbarter Kugeln beziehungsweise der darin befindlichen Aufnahmekanäle exakt dem Kugeldurchmesser entsprechen, so daß in vorteilhafter Weise in allen drei Koordinatenrichtungen von gleichen Abmessungen ausgegangen werden kann.
Gegebenenfalls können insbesondere bei entsprechender Materialauswahl der Trägermatte die Aufnahmekanäle selbst als Führungen für die Strahlenquelle beziehungsweise die mit dem Afterloading-Gerät verbundenen Hohlsonden ausgebildet sein. Separate Führungshülsen wären in diesem Falle nicht mehr erforderlich. Bei dieser Ausführungsform können an einem Ende der Führungs- und Aufnahmekanäle Kupplungen für die Hohlsonden vorgesehen sein.
Weiterhin besteht die Möglichkeit, daß die Trägermatte mit beim Herstellen eingelegten Führungshülsen versehen ist. Bei dieser Ausführungsform ist ein nachträgliches Bestücken mit Führungshülsen nicht mehr erforderlich.

Eine Weiterbildung der Erfindung sieht vor, daß bei der Trägermatte und/oder den Führungshülsen Strahlen-Abschirmelemente an deren Rückseite angeordnet sind, daß eine unerwünschte rückseitige Abstrahlung unterdrückt wird.
Durch diese Abschirmelemente kann eine unerwünschte, rückseitige Abstrahlung vermieden oder zumindest reduziert werden.
Dabei ist bevorzugt vorgesehen, daß an den Abschirmelementen Halterungen zum Verbinden mit der Trägermatte vorgesehen sind, die vorzugsweise zum Eingreifen in Durchbrüche der Trägermatte insbesondere pilzartig ausgebildet sind. Diese Abschirmungen können bedarfsweise ganz gezielt dort angebracht werden, wo die Strahlenquelle entlangbewegt wird.
Durch die vorgesehenen Halter ist eine schnelle Montage und auch Demontage beziehungsweise ein Umsetzen der Abschirmelemente möglich.
Vorteilhaft ist es, wenn die Abschirmelemente streifen- oder plättchenförmig ausgebildet sind und sich jeweils zumindest über den Bereich einer Kugel und gegebenenfalls an diese angrenzende Halsabschnitte erstreckt.
Dadurch bleibt die gute Formanpaßbarkeit der Trägermatte weitestgehend unbeeinflußt.
Gegebenenfalls können die Führungshülsen zumindest bereichsweise Strahlen-Abschirmungen aufweisen, die einen Umfangsabschnitt der Führungshülse überdecken. Somit ist bereits nahe an der Strahlenquelle eine Abschirmung vorhanden, die dann bedarfsweise in Kombination mit einer Abschirmung an der Trägermatte eine besonders wirksame Abschirmung ergibt, die sonst erforderliche separate Abschirmungen meist überflüssig macht.

Zusätzliche Ausgestaltungen der Erfindung sind in den weiteren Unteransprüchen aufgeführt. Nachstehend ist die Erfindung mit ihren wesentlichen Einzelheiten anhand der Zeichnungen noch näher erläutert.

Es zeigt:
- Fig. 1: eine perspektivische Ansicht eines Teilabschnittes einer Trägermatte und schematisch angedeutetem Afterloading-Gerät,
- Fig. 2: einen Teilabschnitt einer Trägermatte mit unterschiedlichen darin befindlichen Aufnahmekanälen
- Fig. 3 und 4: Schmalseitenansichten von Trägermatten mit verschiedenen Aufnahmekanälen,
- Fig. 5: eine perspektivische Teilansicht einer Trägermatte mit Strahlen-Abschirmelementen,
- Fig. 6: eine Seitenansicht von mehreren Strahlen-Abschirmelementen,
- Fig. 7: einen Teilabschnitt einer Trägermatte mit kachelförmigen Strahlen-Abschirmelementen,
- Fig. 8 und 9: Führungshülsen mit bereichsweise am Umfangsabschnitt angeordneten Strahlen-Abschirmungen,
- Fig. 10 bis 13: Seitenansichten von Trägermatten mit unterschiedlich profilierten Aufnahmekanälen und
- Fig. 14: eine Aufsicht eines Teilabschnittes einer Trägermatte mit Kenn-Codierung der einzelnen Aufnahmekanal-Reihen.

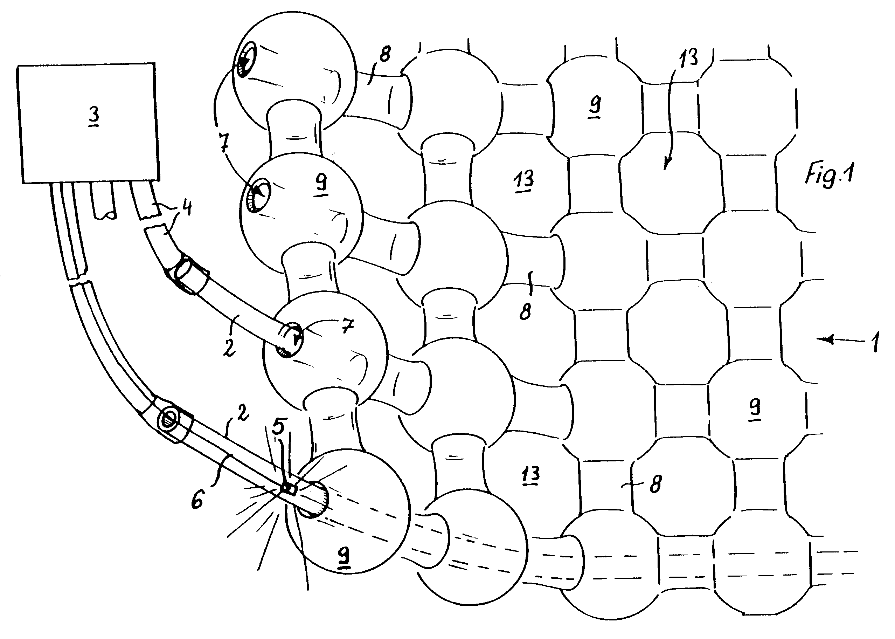

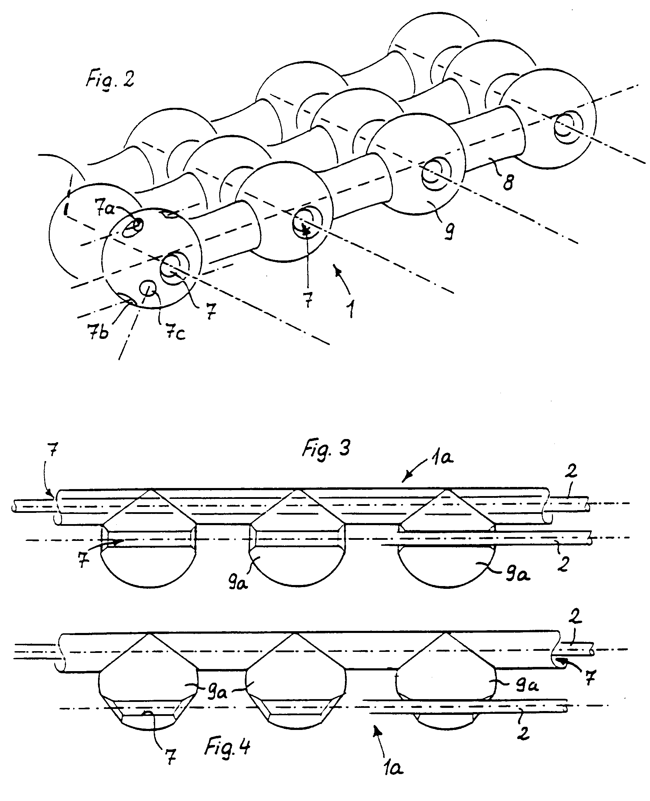

Eine in Figur 1 gezeigte Trägermatte 1 dient im Ausführungsbeispiel zur Aufnahme von Führungshülsen 2. Die Trägermatte dient zur medizinischen Strahlentherapie und wird im Behandlungsbereich an der zu bestrahlenden Stelle positioniert. In die Führungshülsen 2 wird dazu nacheinander eine im wesentlichen punktförmige Strahlenquelle, bestehend aus einem radioaktiven Präparat, in die Führungshülsen 2 eingeführt und im Verlauf dieser Führungshülsen für genau vorausberechnete Zeitabschnitte positioniert. Die Strahlenquelle wird dann aus der einen Führungshülse herausgezogen und in eine andere Führungshülse der Trägermatte eingeführt und dort positioniert.
Die Führungshülsen oder Applikatoren, in die das Strahlen-Präparat eingeführt werden soll, sind mit zu einem Afterloading-Gerät 3 führenden Hohlsonden 4 kuppelbar. Das Afterloading-Gerät 3 beinhaltet einen Strahlenschutzbehälter für die Strahlenquelle 5 in den diese nach der jeweiligen Behandlung an einem Transportdraht 6 zurückgezogen werden kann. Das Afterloading-Gerät 3 ist so programmierbar, daß die Strahlenquelle 5 nacheinander in die jeweiligen Führungshülsen 2 eingeführt und im Bereich der Trägermatte 1 an bestimmten Stellen auch positioniert wird.
Vor einer Behandlung wird die Trägermatte 1 entsprechend der Größe und Lage des Behandlungsfeldes mit Führungshülsen 2 bestückt. Die Trägermatte 1 ist dazu mit rasterförmig nebeneinander angeordneten Aufnahmekanälen 7 versehen. In diese werden die Führungshülsen 2 direkt eingeführt.
Spezielle Hilfselemente, wie beispielsweise Stahlmandrins, die beim Einstechen von nadelförmigen Führungshülsen in eine nicht mit Aufnahmekanälen 7 versehene Trägermatte bisher notwendig waren, sind nicht erforderlich.
Die Führungshülsen 2 sind durch die bereits in der Trägermatte 1 enthaltenen Aufnahmekanäle 7 exakt in einem vorgegebenen Verlauf geführt, so daß auch eine entsprechend exakte Vorausberechnung der Strahlendosis beziehungsweise der jeweiligen Verweildauer der Strahlenquelle 5 an bestimmten Stellen möglich ist.
Durch die Trägermatte 1, die auch als Moulage bezeichnet wird, ist ein genau vorgegebener Abstand der Strahlenquelle von der Oberfläche des zu behandelnden Bereiches gegeben.
Die Trägermatte 1 kann beispielsweise eine Dicke von etwa 10mm aufweisen, um bei Einsatz üblicher Strahler ein homogenes Strahlenfeld und eine homogene Strahlendosis zu erreichen.
Um nun die Lage der in der Trägermatte 1 eingeführten Führungshülsen 2 und damit auch die Lage der später eingeführten strahlenquelle 5 variieren zu können, kann die Trägermatte 1 auch außerhalb ihrer Mittelebene angeordnete Aufnahmekanäle 7 aufweisen. In Figur 1 sind die Aufnahmekanäle 7 etwa in der Mittelebene angeordnet und Figur 2 zeigt eine Ausführungsform einer Trägermatte, bei der zusätzlich noch außermittig verlaufende Aufnahmekanäle 7a, 7b vorgesehen sind. Diese Aufnahmekanäle sind in Figur 2 nur an einem der aus Kugelementen gebildeten Trägermatte 1 gezeigt. Sie setzen sich aber jeweils in Fluchtrichtung auch in den jeweils benachbarten Kugelkörpern fort, so wie dies in Figur 3 und 4 erkennbar ist.
Weiterhin besteht die Möglichkeit, auch in Querrichtung verlaufende Aufnahmekanäle 7c vorzusehen (Figur 2).

Die Trägermatte 11 kann im wesentlichen als Flachkörper aus gut verformbarem, anschmiegsamen Kunststoff-Material bestehen. Um eine besonders gute Beweglichkeit und Verformbarkeit der Trägermatte auch bei einer etwas dickeren Ausführung beispielsweise von 10mm oder mehr zu erzielen ist vorgesehen, daß die Trägermatte aus einer Vielzahl von nebeneinander angeordneten, miteinander über Halsabschnitte 8 verbundene Kugeln 9 oder ähnliche, gerundete Körper 9a besteht. Bevorzugt ist die Ausführungsform beispielsweise gemäß Figur 1 und 2. Die Aufnahmekanäle 7 laufen hier sowohl durch die Kugeln 9 als auch durch die Halsabschnitte 8.
Figur 2 zeigt darüberhinaus noch die weiteren Aufnahmekanäle 7a und 7c, die nur durch die Kugeln 9 verlaufen. Da aber bei einer zur Behandlung vorbereiteten Trägermatte 1 die Führungshülsen 2 eingeführt sind, ist für die Strahlenquelle 5 innerhalb der in die Aufnahmekanäle 7a oder 7b oder 7c eingesetzten Führungshülsen 2 eine durchgehende Führung vorhanden. Die Trägermatte 1 gemäß Figur 1 und 2 ist bezüglich der Mittelebene symmetrisch aufgebaut und weist dadurch eine gleich gute Verformbarkeit und Bewegbarkeit in beide Richtungen auf.
Es ist aber auch eine unsymmetrische Ausführungsform der Trägermatte 1a gemäß Figur 3 oder Figur 4 möglich. Hierbei liegen die Verbindungs-Halsabschnitte 8 nicht in der Mittelebene der gerundeten Körper 9a sondern zu einer Oberflächenseite hin versetzt.
Der Durchmesser der Kugeln 9 beziehungsweise der gerundeten Körper 9a, der Querschnitt der Halsabschnitte 8 und auch die Abstände zwischen den Kugeln beziehungsweise den Halsabschnitten kann je nach den Erfordernissen variiert werden und wird insbesondere auf die erforderlichen Abstandsverhältnisse und die Beweglichkeit der Trägermatte abgestimmt.

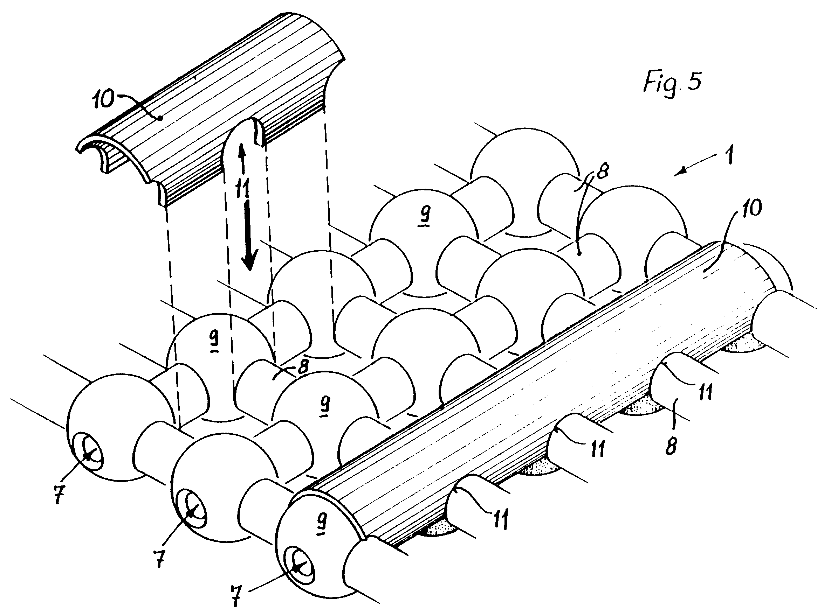

Um eine Rundumabstrahlung von der Strahlenquelle 5 zu vermeiden und insbesondere das Bestrahlen und eventuelle Schädigen von außerhalb des Behandlungsbereiches befindlichem Gewebe zu verhindern, können zur Abschirmung unterschiedliche Maßnahmen vorgesehen sein, wie dies insbesondere in Figur 5 bis 9 gezeigt ist. Figur 5 zeigt dabei etwa schalenförmige Strahlen-Abschirmelemente 10, die entlang von den in der Trägermatte 1 befindlichen Aufnahmekanälen 7 anbringbar sind. Zum Verbinden dieser Abschirmelemente 10 mit der Trägermatte 1 sind an den Abschirmelementen Rastausnehmungen 11 vorgesehen, die jeweils beidseitig einer Kugel 9 bei den Halsabschnitten 8 eingreifen. Der lichte Durchmesser der Rastausnehmungen 11 entspricht etwa dem Durchmesser der Halsabschnitte 8, wobei die Rastausnehmungen sich etwas über einen Halbkreis erstrecken, so daß die unteren freien Ränder die Halsabschnitte 8 rastend etwas übergreifen. Dadurch können die Abschirmelemente 10 an den jeweils vorgesehenen Stellen eingeklippst werden. Bevorzugt wird die Abschirmung von einer Vielzahl von vergleichsweise kurzen Abschirmelementen 10 erstellt, damit die Beweglichkeit der Trägermatte 1 weitestgehend erhalten bleibt. Diese Abschirmelemente erstrecken sich deshalb vorzugsweise nur über den Bereich einer Kugel 9 und gegebenenfalls die angrenzenden Halsabschnitte 8. Für Anwendungsfälle, wo jedoch die Trägermatte 11 eben angeordnet ist, oder nur in einer Ebene verformt wird, können auch längere Abschirmelemente 10 eingesetzt werden.

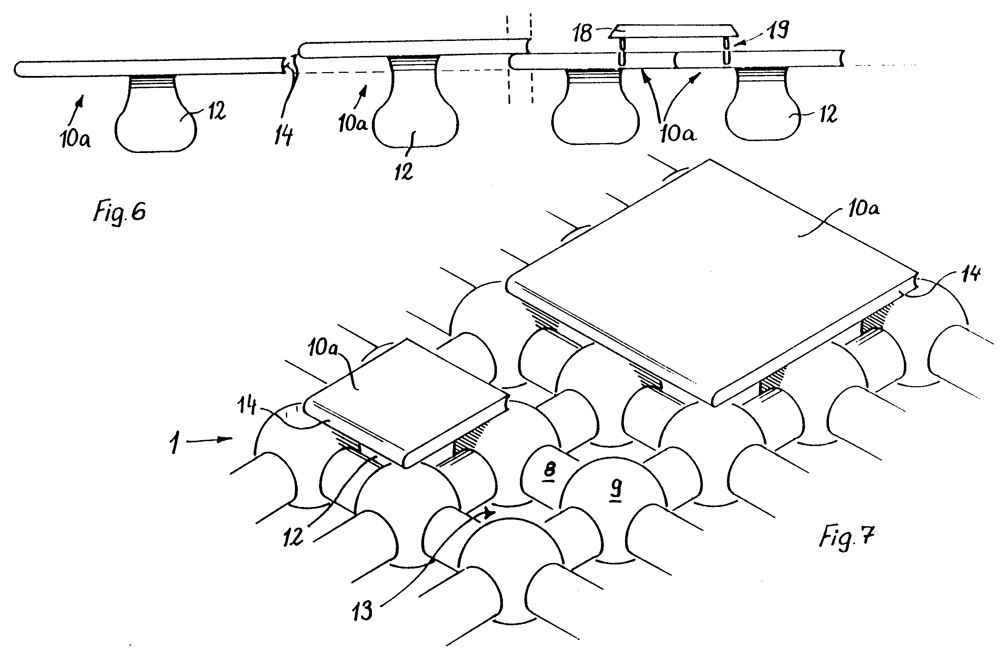

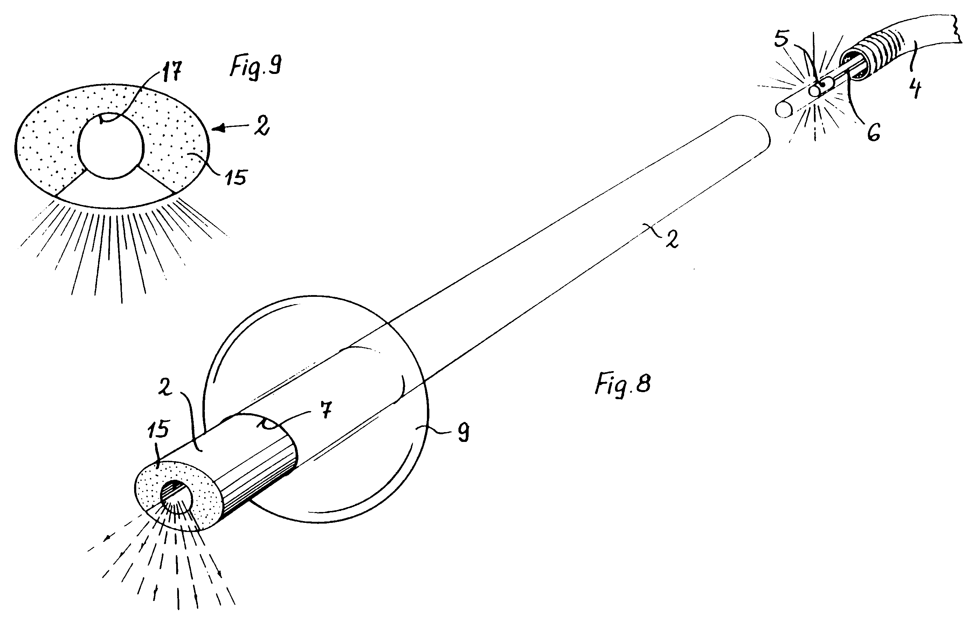

Die Figuren 6 und 7 zeigen etwa kachelartige Abschirmelemente 10a, die etwa pilzartige Rastelemente 12 zum Einklippsen in die Durchbrüche 13 der Trägermatte 1, 1a aufweisen. Die Abschirmelemente 10a - auch die Abschirmelemente 10 - können randseitige Eingriffsprofilierungen 14 aufweisen, so daß benachbarte Abschirmelemente randseitig etwas ineinandergreifen und so ein weitgehend strahlendichter Übergang geschaffen ist (Figur 6). Es besteht aber auch die Möglichkeit, daß die Abschirmelemente so ausgebildet sind, daß sich eine dachziegelartige Überlappung ergibt, wie dies ebenfalls in Figur 6 dargestellt ist. Zur Überdeckung der Trennbereiche zwischen benachbarten Abschirmelementen 10a können abschirmende Abdeckelemente 18 (Figur 6) vorgesehen sein.
Diese lassen sich beispielsweise über Steckrastverbindungen 19 an den Abschirmelementen 10a anbringen.
Auch die kachelförmigen Abschirmelemente 10a gemäß Figur 6 und 7 können in unterschiedlichen Größen vorgesehen sein.
Bei kleinen Abschirmelementen kann zentral mittig ein einziges Rastelement vorgesehen sein, währen bei größerflächigen Abschirmelementen auch mehr als ein Rastelement 12 vorgesehen sein kann, um eine sichere Halterung zu gewährleisten.

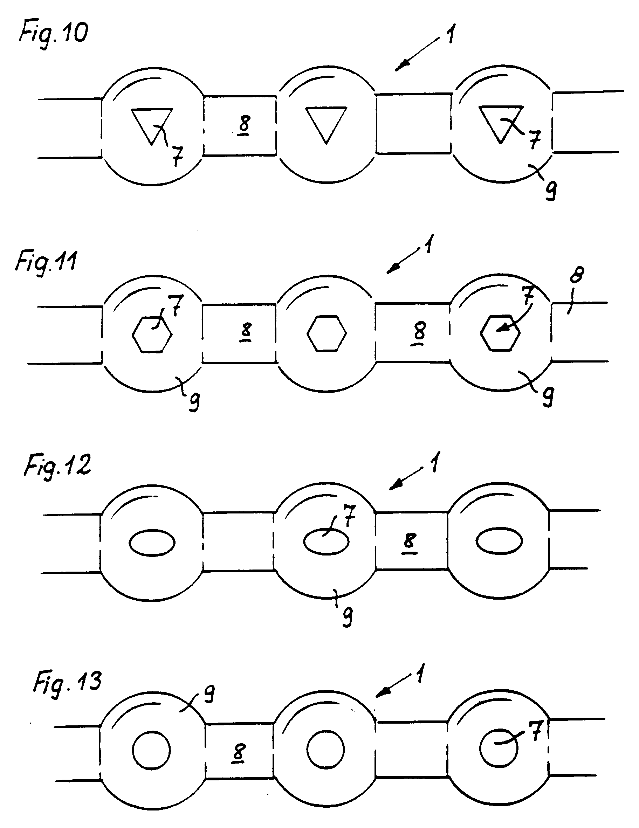

Die Figuren 8 und 9 zeigen weitere Möglichkeiten von Strahlen-Abschirmmaßnahmen. In diesem Falle befindet sich die Abschirmung 15 direkt bei den Führungshülsen 2. Bei dieser Ausführungsform besteht ein Umfangsteil der die Innenhöhlung 17 umgrenzenden Wandung der Führungshülsen 2 aus Abschirmmaterial, wobei die Größe des Umfangsabschnittes gleichzeitig auch den möglichen Abstrahlwinkel auf der gegenüberliegenden Seite mitbestimmt. Zur Lagefixierung solcher Abschirm-Führungshülsen innerhalb der Aufnahmekanäle 7 weisen die Führungshülsen 2 und die Aufnahmekanäle 7 in der Trägermatte 1 ineinanderpassende Längsprofilierungen auf. Im Ausführungsbeispiel gemäß Figur 8 und 9 und auch in Figur 12 ist eine ovale Querschnittsform vorgesehen. Es können aber auch andere Querschnittsprofilierungen vorgesehen sein, wie dies in Figur 10 und 11 gezeigt ist.
Darüberhinaus lassen sich auch in Umfangsrichtung unsymmetrische Profilierungen einsetzen, durch die dann ein Einsetzen der Führungshülsen 2 nur in einer einzigen passenden Lage möglich ist.
Die einzelnen Abschirmmaßnahmen wie sie in Figur 8 und 9 beziehungsweise in Figur 6 und 7 oder Figur 5 gezeigt sind, lassen sich auch in Kombination miteinander einsetzen.
Erwähnt sei noch, daß zur Abschirmung der in den Führungsröhrchen 2 geführten Strahlenquelle 5 auch auf diese Führungshülsen 2 aufschiebbare, längsgeschlitzte Abschirmhülsen vorgesehen sein könnten. Diese Abschirmhülsen hätten dann etwa einen Umfangsverlauf wie die in Figur 9 gezeigte Abschirmung 15.

Bevorzugt ist vorgesehen, daß die mit Aufnahmekanälen versehene Trägermatte vor dem Gebrauch mit Führungshülsen 2 bestückt wird. Dies ist deshalb vorteilhaft, weil bisher bereits vorhandenen Führungshülsen 2 verwendet werden können. Es besteht aber auch die Möglichkeit, daß solche Führungshülsen 2 gleich bei der Herstellung der Trägermatte mit eingelegt werden, so daß die Matte dann praktisch voll bestückt dem Anwender zur Verfügung steht. Weiterhin könnten die Aufnahmekanäle 7 selbst auch so ausgebildet sein, daß sie selbst Führungen für die Strahlenquelle bilden. In diesem Falle wären dann direkt an der Trägermatte bei den Austrittsenden der Aufnahmekanäle 7 Anschlüsse zum Kuppeln mit den Hohlsonden 4 des Afterloading-Gerätes 3 vorgesehen.
Die in den Ausführungsbeispielen gezeigte Form der Trägermatte mit einer gitterartigen Struktur vereinfacht auch das Zuschneiden der Trägermatte, da hier rasterartig Trennlinien vorgegeben sind.

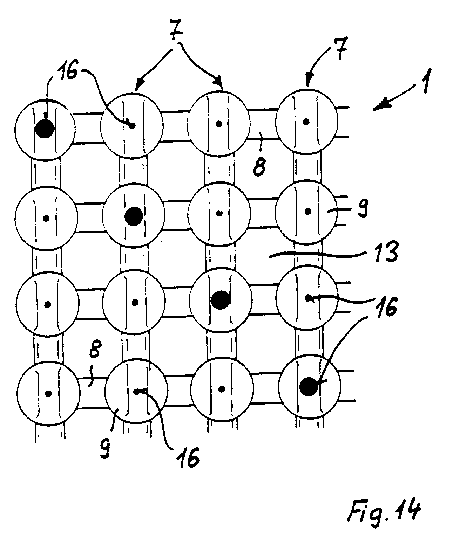

Um die Trägermatte gut auf einem Röntgenschirm darstellen zu können und damit ihre Lage zu kontrollieren, sind im Verlauf der Aufnahmekanäle röntgendichte Materialeinschlüsse 16 vorgesehen. Im vorliegenden Ausführungsbeispiel weist jede Kugel 9 der Trägermatte 1 einen solchen Materialeinschluß 16 auf. Um die einzelnen Aufnahmekanäle der Trägermatte auch auf dem Röntgenbild gut voneinander unterscheiden zu können, können die Materialeinschlüsse in Form einer Kodierung positioniert beziehungsweise ausgebildet sein.
In dem in Figur 14 gezeigten Ausführungsbeispiel ist die Kodierung so vorgenommen, daß in der ersten Reihe an erster Stelle, in der zweiten Reihe an zweiter Stelle usw. jeweils ein etwas größerer Materialeinschluß vorgesehen ist. Die Kodierung kann aber in vielfältigen anderen Ausführungsformen vorgesehen sein.
Weiterhin besteht die Möglichkeit, daß die Trägermatte mit vorzugsweise rasterförmig verteilt angeordheten Lagesensoren versehen ist, die mit einem Computer verbunden sind. Es ist dadurch eine direkte Lagedarstellung der Trägermatte auf dem Computer-Bildschirm möglich. Durch diese Lagesensoren kann eine wesentlich exaktere Dosisberechnung unter Einbeziehung der Formung der Matte und den darin geführten Strahlern durchgeführt werden. Es ist dadurch auch eine wesentlich exaktere Berechnung der Verweildauer der Strahler an den jeweiligen Bestrahlungsstellen möglich.

Die Matte kann auch zur Behandlung äußerlicher Bereiche, zum Beispiel von Oberflächentumoren, eingesetzt werden. Sie wird dazu auf den zu behandelnden Bereich von außen aufgelegt.
Es besteht weiterhin die Möglichkeit, in die Führungskanäle verformbare Versteifungselemente einzubringen, die dann dafür sorgen daß nach einem Formanpassen der Matte diese Form beibehalten bleibt. Dadurch läßt sie sich bei mehrmaligem Gebrauch immer wieder gut positionieren.

## Patentansprüche

1. Vorrichtung für die medizinische Strahlentherapie mit einer im Behandlungsbereich beim Patienten anbringbaren, verformbaren Trägermatte (1, 1a) für Führungshülsen (2), in denen eine im wesentlichen punktförmige Strahlenquelle (5) verschiebbar und positionierbar ist, wobei die Trägermatte (1, 1a) innerhalb ihrer Mattenebene verlaufende, vorgefertigte Aufnahmekanäle (7, 7a, 7b, 7c) zur Aufnahme der Führungshülsen (2) oder dergleichen aufweist, **dadurch gekennzeichnet**, daß die Trägermatte einstückig aus Silikon oder einem silikonähnlichen, autoklavierbaren Material besteht, daß die Trägermatte aus einer Vielzahl von nebeneinander angeordneten, miteinander über Halsabschnitte (8) verbundenen Kugeln (9) oder gerundeten, kugelähnlichen, ballförmigen Körpern (9a) gebildet ist und daß die Aufnahmekanäle durch die Kugeln (9) und/oder die Halsabschnitte (8) verlaufen.

2. Vorrichtung nach Anspruch 1, dadurch gekennzeichnet, daß die Aufnahmekanäle in einem vorgebbaren Raster angeordnet sind und daß vorzugsweise die Aufnahmekanäle parallel nebeneinander mit insbesondere gleichen Seitenabständen zueinander angeordnet sind.

3. Vorrichtung nach Anspruch 1 oder 2, dadurch gekennzeichnet, daß zumindest ein Teil der Aufnahmekanäle bezüglich der zu den Flachseiten der Trägermatte parallel verlaufenden Mittelebene versetzt angeordnet sind.

4. Vorrichtung nach Anspruch 3, dadurch gekennzeichnet, daß mehrere Aufnahmekanäle in unterschiedlichen Ebenen übereinander angeordnet sind.

5. Vorrichtung nach einem der Ansprüche 1 bis 4, dadurch gekennzeichnet, daß Aufnahmekanäle winklig zueinander verlaufend angeordnet sind.

6. Vorrichtung nach einem der Ansprüche 1 bis 5, dadurch gekennzeichnet, daß die Trägermatte Durchbrüche (13) vorzugsweise zwischen den Aufnahmekanälen (7) beziehungsweise den Kugeln (9) aufweist.

7. Vorrichtung nach Anspruch 6, dadurch gekennzeichnet, daß die Durchbrüche rasterartig über die Trägermatte verteilt angeordnet sind.

8. Vorrichtung nach einem der Ansprüche 1 bis 7, dadurch gekennzeichnet, daß die Kugeln zueinander beabstandet sind und durch die halsförmige Zwischenelemente (8) miteinander verbunden sind.

9. Vorrichtung nach einem der Ansprüche 1 bis 8, dadurch gekennzeichnet, daß die Kugeln (9) an ihren einander zugewandten Seiten im Bereich der Verbindungsstellen mit den Halsabschnitten (8) jeweils um die halbe Länge der Halsabschnitte abgeflacht sind und daß der Mittenabstand benachbarter Kugeln ihrem Durchmesser entspricht.

10. Vorrichtung nach einem der Ansprüche 1 bis 9, dadurch gekennzeichnet, daß die Aufnahmekanäle selbst als Führungen für die Strahlenquelle beziehungsweise die mit einem Afterloading-Gerät (3) verbundenen Hohlsonden (4) ausgebildet sind.

11. Vorrichtung nach einem der Ansprüche 1 bis 10, dadurch gekennzeichnet, daß bei der Trägermatte und/oder den Führungshülsen (2) Strahlen-Abschirmelemente (10, 10a, 15) derart an deren Rückseite angeordnet sind, daß eine unerwünschte rückseitige Abstrahlung unterdrückt wird.

12. Vorrichtung nach Anspruch 11, dadurch gekennzeichnet, daß an den Abschirmelementen Halterungen zum Verbinden mit der Trägermatte vorgesehen sind, die vorzugsweise zum Eingreifen in Durchbrüche (13) der Trägermatte insbesondere pilzartig ausgebildet sind.

13. Vorrichtung nach Anspruch 11 oder 12, dadurch gekennzeichnet, daß die Abschirmelemente streifen- oder plättchenförmig ausgebildet sind und sich jeweils zumindest über den Bereich einer Kugel (9) und gegebenenfalls an diese angrenzende Halsabschnitte (8) erstreckt.

14. Vorrichtung nach einem der Ansprüche 11 bis 13, dadurch gekennzeichnet, daß benachbarte Abschirmelemente randseitig Eingriffsprofilierungen aufweisen und/oder dachziegelartig sich überlappend angeordnet sind.

15. Vorrichtung nach einem der Ansprüche 11 bis 14, dadurch gekennzeichnet, daß die Führungshülsen (2) zumindest bereichsweise Strahlen-Abschirmungen (15) aufweisen, die einen Umfangsabschnitt der Führungshülse überdecken.

16. Vorrichtung nach einem der Ansprüche 1 bis 15, dadurch gekennzeichnet, daß die Führungshülsen (2) und die Aufnahmekanäle (7) in der Trägermatte ineinanderpassende Längsprofilierungen als Verdrehsicherung aufweisen.

17. Vorrichtung nach einem der Ansprüche 11 bis 16, dadurch gekennzeichnet, daß als Abschirmung für die Führungshülsen längsgeschlitzte, auf die Führungshülsen (2) aufschiebbare Abschirmhülsen vorgesehen sind.

18. Vorrichtung nach einem der Ansprüche 1 bis 17, dadurch gekennzeichnet, daß im Verlauf der Aufnahmekanäle röntgendichte Materialeinschlüsse vorgesehen sind, die vorzugsweise bezüglich der Lage des jeweiligen Aufnahmekanales codiert sind.

19. Vorrichtung nach einem der Ansprüche 1 bis 18, dadurch gekennzeichnet, daß die Trägermatte mit beim Herstellen eingelegten Führungshülsen (2) versehen ist.

20. Vorrichtung nach einem der Ansprüche 1 bis 19, dadurch gekennzeichnet, daß die Trägermatte mit vorzugsweise rasterförmig verteilt angeordneten Lagesensoren versehen ist, die an einen Rechner mit Bildschirm anschliessbar sind.

## Claims

1. Device for medicinal radiation therapy, comprising a deformable support mat (1, 1a) for guide tubes (2), which can be applied to a treatment region at the patient, in which guide tubes a substantially punctiform radiation source (5) can be displaced and positioned, the support mat (1, 1a) possessing prefabricated seating channels (7, 7a, 7b, 7c) running within the plane of the mat for receiving the guide tubes (2) or the like, characterized in that the support mat consists in one piece of silicone or a silicone-like material which can be autoclaved, that the support mat is formed of a plurality of balls (9) or rounded, ball-like objects (9a), arranged alongside one another and connected to one another by tube sections (8), and that the seating channels pass through the balls (9) and/or the tube sections (8).

2. Device according to Claim 1, characterized in that the seating channels are arranged in a predeterminable grid and that preferably the seating channels are disposed parallel to one another, especially with equal lateral spacings from one another.

3. Device according to Claim 1 or 2, characterized in that at least a portion of the seating channels are disposed offset from the central plane extending parallel to the flat faces of the support mat.

4. Device according to Claim 3, characterized in that several seating channels are disposed above one another in different planes.

5. Device according to one of Claims 1 to 4, characterized in that seating channels are disposed at angles to one another.

6. Device according to one of Claims 1 to 5, characterized in that the support mat possesses apertures (13) preferably between the seating channels (7) and/or the balls (9).

7. Device according to Claim 6, characterized in that the apertures are arranged in a grid distributed across the support mat.

8. Device according to one of Claims 1 to 7, characterized in that the balls are spaced apart from one another and are connected together by the tubular intermediate elements (8).

9. Device according to one of Claims 1 to 8, characterized in that the balls (9) are flattened at their mutually facing sides in the region of the connecting locations to the tube sections (8), each by one-half of the length of the tube section, and that the centre-to-centre distance between adjacent balls corresponds to their diameter.

10. Device according to one of Claims 1 to 9, characterized in that the seating channels themselves are formed as guides for the radiation source or the hollow probes (4) connected to an after-loading device (3).

11. Device according to one of Claims 1 to 10, characterized in that, at the support mat and/or the guide tubes (2), radiation shielding elements (10, 10a, 15) are disposed in such a way on their rear side that an undesired emission of radiation from the rear side is suppressed.

12. Device according to Claim 11, characterized in that holders for connecting to the support mat are provided at the shielding elements, which holders are shaped, especially mushroom-shaped, for engaging into apertures (13) of the support mat.

13. Device according to Claim 11 or 12, characterized in that the shielding elements are constructed strip-shaped or platelet-shaped and each extends at least over the region of one ball (9) and optionally also over tube sections (8) adjacent thereto.

14. Device according to one of Claims 11 to 13, characterized in that adjacent shielding elements possess engagement profiles at their edges and/or are arranged to overlap in the manner of roof tiles.

15. Device according to one of Claims 11 to 14, characterized in that the guide tubes (2) possess, at least in regions, radiation shields (15), which cover over a circumferential portion of the guide tube.

16. Device according to one of Claims 1 to 15, characterized in that the guide tubes (2) and the seating channels (7) possess longitudinal profiles fitting into the support mat, as a prevention against rotation.

17. Device according to one of Claims 11 to 16, characterized in that longitudinally slit shielding sleeves, which can be pushed onto the guide tubes (2), are provided as shielding for the guide tubes.

18. Device according to one of Claims 1 to 17, characterized in that X-ray dense material inclusions are provided along the path of the seating channels, which inclusions are preferably coded in respect of the position of the particular seating channel.

19. Device according to one of Claims 1 to 18, characterized in that the support mat is provided with guide tubes (2) incorporated in it during manufacture.

20. Device according to one of Claims 1 to 19, characterized in that the support mat is provided with position sensors, preferably distributed in a grid pattern, which can be connected to a computer having a display screen.

## Revendications

1. Dispositif de radiothérapie médicale comportant une natte de support déformable (1, 1a), susceptible d'être appliquée dans la zone de traitement du patient, pour douilles de guidage (2), dans lesquelles une source de rayons (5) sensiblement ponctuelle peut être déplacée et positionnée, la natte de support (1, 1a) présentant des canaux récepteurs (7, 7a, 7b, 7c) préusinés s'étendant dans le plan de la natte pour recevoir les douilles de guidage ou des dispositifs similaires, caractérisé en ce que la natte de support est constituée d'une pièce à partir de silicone ou d'une matière de type silicone susceptible de passer à l'autoclave, en ce que la natte de support est formée d'une série de sphères (9), ou de corps arrondis (9a) en forme de sphères ou de billes, agencées les unes à côté des autres et reliées les unes aux autres par des sections intermédiaires en forme de col et en ce que les canaux récepteurs s'étendent à travers les sphères (9) et les sections intermédiaires (8).

2. Dispositif selon la revendication 1, caractérisé en ce que les canaux récepteurs sont agencés sous la forme d'un réseau prédéterminé et en ce que, de préférence, les canaux récepteurs soient agencés parallèlement les uns à côté des autres avec, en particulier, des distances latérales mutuelles égales.

3. Dispositif selon la revendication 1 ou 2, caractérisé en ce qu'au moins une partie des canaux récepteurs sont agencés décalés par rapport au plan central s'étendant parallèlement aux faces plates de la natte de support.

4. Dispositif selon la revendication 3, caractérisé en ce que plusieurs canaux récepteurs sont agencés les uns au-dessus des autres dans des plans différents.

5. Dispositif selon l'une quelconque des revendications 1 à 4, caractérisé en ce que les canaux récepteurs sont agencés de manière à faire un angle mutuel.

6. Dispositif selon l'une quelconque des revendications 1 à 5, caractérisé en ce que la natte de support présente des interruptions (13), de préférence, entre les canaux récepteurs (7) ou les sphères (9).

7. Dispositif selon la revendication 6, caractérisé en ce que les interruptions sont réparties en réseau sur la natte de support.

8. Dispositif selon l'une quelconque des revendications 1 à 7, caractérisé en ce que les sphères sont distantes les unes des autres et sont reliées les unes aux autres par les éléments intermédiaires (8) en forme de col.

9. Dispositif selon l'une quelconque des revendications 1 à 8, caractérisé en ce que les sphères (9) sont aplaties sur leurs côtés tournés l'un vers l'autre dans la zone des emplacement de liaison avec les sections intermédiaires (8), respectivement, sur la demi-longueur des sections intermédiaires et en ce que la distance aux centres des sphères voisines correspond a leur diamètre.

10. Dispositif selon l'une quelconque des revendications 1 à 9, caractérisé en ce que les canaux récepteurs se présentent eux-mêmes sous la forme de guides pour la source de rayons ou les sondes creuses connectées à un dispositif de stockage et de positionnement de la source de rayons.

11. Dispositif selon l'une quelconque des revendications 1 à 10, caractérisé en ce qu'on agence, dans la natte de support et/ou les douilles de guidage (2), des éléments de protection contre les rayons (10, 10a, 15) sur leur verso de manière à supprimer le rayonnement non souhaité au verso.

12. Dispositif selon la revendication 11, caractérisé en ce qu'on agence sur les éléments de protection des attaches pour assurer la liaison avec la natte de support, lesdites attaches étant conformées de préférence pour s'engager dans des interruptions (13) de la natte de support, en particulier en forme de champignon.

13. Dispositif selon la revendication 11 ou 12, caractérisé en ce que les éléments de protection sont conformés en bandes ou en plaques et s'étendent respectivement au moins sur la zone d'une sphère (9) et éventuellement sur les sections intermédiaires (8) qui sont contiguës à celle-ci.

14. Dispositif selon l'une quelconque des revendications 11 à 13, caractérisé en ce que des éléments de protection voisins présentent des profilage d'engagement sur les bords et/ou sont agencés à chevauchement à la façon des tuiles d'un toit.

15. Dispositif selon l'une quelconque des revendications 11 à 14, caractérisé en ce que les douilles de guidage (2) présentent au moins dans certaines parties des écrans protecteurs contre les rayons (15), qui recouvrent une section périphérique de la douille de guidage.

16. Dispositif selon l'une quelconque des revendications 1 à 15, caractérisé en ce que les douilles de guidage (2) et les canaux récepteurs (7) présentent des profilages longitudinaux s'adaptant l'un dans l'autre dans la natte de support comme sécurité contre la torsion.

17. Dispositif selon l'une quelconque des revendications 11 à 16, caractérisé en ce qu'il est prévu comme protection pour les douilles de guidage des douilles de protection longitudinalement divisées, emboîtables sur les douilles de guidage (2).

18. Dispositif selon l'une quelconque des revendications 1 à 17, caractérisé en ce qu'il est prévu dans le trajet des canaux récepteurs des inclusions de matière étanche aux rayons X, qui sont codées de préférence par rapport à la position du canal récepteur respectif.

19. Dispositif selon l'une quelconque des revendications 1 à 18, caractérisé en ce que la natte de support est pourvue de douilles de guidage (2) insérées lors de la fabrication.

20. Dispositif selon l'une quelconque des revendications 1 à 19, caractérisé en ce que la natte de support est pourvue de capteurs de position répartis de préférence en forme de réseau, qui peuvent être connectés à un ordinateur pourvu d'un écran.
